# EUROPEAN PATENT SPECIFICATION

(11) **EP 4 338 713 B1**
(45) Date of publication and mention of the grant of the patent: **26.11.2025**
(21) Application number: 22827384.3
(22) Date of filing: 08.06.2022
(51) Int. Cl.: A61F 2/82, A61M 29/02

(54) **STENT AND MEDICINE CARRYING STENT**
STENT UND ARZNEIMITTELTRAGENDER STENT
STENT ET STENT DE TRANSPORT DE MÉDICAMENTS

(30) Priority: 22.06.2021 CN 202110693999
(43) Date of publication of application: 20.03.2024
(73) Proprietor: MicroPort NeuroTech (Shanghai) Co., Ltd., Shanghai 201318 (CN)
(72) Inventor: SONG, Zhihao, Shanghai 201318 (CN); HOU, Juan, Shanghai 201318 (CN); TIAN, Hao, Shanghai 201318 (CN); LEI, Wenbin, Shanghai 201318 (CN)
(74) Representative: Marks & Clerk LLP
(86) International application number: PCT/CN2022/097717
(87) International publication number: WO 2022/267888

(56) References cited:
- EP-A1- 2 210 573
- EP-A1- 2 210 573
- CN-A- 101 642 397
- CN-A- 102 451 051
- CN-A- 102 525 701
- CN-A- 103 945 797
- CN-A- 107 569 311
- CN-A- 108 261 275
- CN-C- 100 471 470
- CN-Y- 2 708 927
- CN-Y- 201 135 513
- US-A1- 2004 225 350
- US-A1- 2007 288 082
- US-B2- 8 636 792

## Description

### TECHNICAL FIELD

The present invention pertains to the field of medical devices, in particular, it relates to a stent and a drug-loaded stent.

### BACKGROUND

Intracranial atherosclerotic stenosis (ICAS) is a significant cause of the occurrence, development and recurrence of ischemic stroke. Clinical intervention focuses on ICAS cases with symptoms, and principal therapeutic measures include antiplatelet medication, angioplasty and traditional surgery. Although antiplatelet medication is a classic therapy for symptomatic ICAS, it is associated with a high incidence of stroke. Traditional surgery is highly risky and therefore seldom employed.

As a result of developments in intracranial intervention, currently, angioplasty has become an important method for symptomatic ICAS treatment. Angioplasty procedures include dilation with a bare metal stent, a simple balloon, a coronary drug-coated stent or the like. However, bare metal stent or simple balloon based dilation is associated with a risk of restenosis, and coronary drug-coated stents are substantially balloon-expanded drug-coated stents. Such stents have a great outer diameter dimension when loaded in a delivery system, which makes delivery to a target site difficult, in particular through a tortuous vascular site or complex lesion site. In contrast, self-expanding stents designed for delivery by a microcatheter can be more easily delivered to a target site because of a reduced outer diameter dimension when loaded in a delivery system.

Differing from extracranial arteries, the physiological structure of an intracranial artery is characterized by: 1) great physiological curvature, as well as significant physiological tortuosity, in particular when it is arteriosclerotic usually as a consequence of stenosis; and 2) a thin and inelastic outer layer, which is located in the subarachnoid space without support from tissue and therefore exhibits poor resistance to mechanical damage. Once a drug escapes from an intracranial artery through a rupture in its wall, or permeates therethrough, it tends to spread through the cerebrospinal fluid across the entire brain and even reach the spinal cord. Accordingly, stents for use in intracranial arteries are desired to have suitable radial strength. A stent with excessive radial strength may be difficult to deliver to a target site, or may cause damage to a blood vessel during use. On the other hand, a stent with insufficient radial strength may be not able to adequately support a stenotic plaque in a blood vessel. Additionally, in order to be able to cover a plaque in a desired manner, a stent is required to have sufficient metal coverage. However, for an intracranial blood vessel, which is vulnerable and lacks elasticity, high metal coverage tends to cause an excessive radial strength, which may lead to damage to the intracranial blood vessel and hence possible in-stent restenosis. It has been reported that some existing self-expanding stents with excessive radial strength frequently cause damage to intracranial blood vessels. There are also some existing self-expanding stents incapable of providing good therapeutic efficacy due to insufficient metal coverage despite their excellent radial strength.

Thus, these existing stents fail to provide both sufficient metal coverage and desirable radial strength.

EP2210573A1 discloses a nested stent in a body lumen, which is formed with a plurality of stent struts connected each other, the adjacent stent struts are nested each other when the stent is pressed and held, the said stent struts are provided with some interlace-arranged segments, the segments on the adjacent stent struts are formed mesh shape and nested each other. CN100471470C discloses a microporous blood vessel stent for eluting medicaments with a microcellular structure that improves drug loading and designed to enable controlled drug release.

### SUMMARY OF THE INVENTION

It is an object of the present invention to provide a stent, which can overcome the problem of failing to provide both good metal coverage and desirable radial strength associated with conventional stents.

To this end, a first aspect of the present invention provides a stent comprising at least one stent mesh each comprising a plurality of stent struts sequentially connected circumferentially around the stent mesh, the plurality of the stent struts sequentially connected end to end, and a joint formed at the connected ends of adjacent stent struts, the stent mesh configured to expand or collapse as a result of widening or narrowing of angles at the joints,
wherein the stent strut comprises at least one main section and at least one broadened section alternately arranged with the at least one main section, the main section having a width smaller than a width of the broadened section; in each stent mesh, the broadened sections of adjacent stent struts are staggered along an axis of the stent mesh; a radial strength of the stent ranges from 1 kPa to 300 kPa, and when the stent is in an expanded configuration with a diameter of 2.0 mm to 5.0 mm, the radial strength of the stent ranges from 1 kPa to 30 kPa.

Optionally, when the angle at each joint is minimized, the radial strength of the stent may range from 50 kPa to 300 kPa.

Optionally, when the angle at each joint is minimized, the radial strength of the stent may range from 100 kPa to 200 kPa.

Optionally, when the stent is in the expanded configuration with the diameter of 2.0 mm to 5.0 mm, the radial strength of the stent may range from 1 kPa to 15 kPa.

Optionally, when the stent is in the expanded configuration with the diameter of 2.0 mm to 5.0 mm, the radial strength of the stent may range from 1 kPa to 12 kPa.

Optionally, the angle at each joint may range from 0° to 140°.

Optionally, when the angle at each joint is in the range of 0° to 5°, metal coverage of the stent mesh may range from 30% to 99%.

Optionally, when the angle at each joint is in the range of 5° to 30°, a metal coverage of the stent mesh may range from 5% to 90%.

Optionally, when the angle at each joint is in the range of 30° to 90°, a metal coverage of the stent mesh may range from 4% to 15%.

Optionally, when the angle at each joint is in the range of 30° to 90°, the metal coverage of the stent mesh may range from 8% to 15%.

Optionally, when the angle at each joint is in the range of 90° to 140°, metal coverage of the stent mesh may range from 3% to 12%.

Optionally, when the angle at each joint is in the range of 0° to 5°, metal coverage of the stent may range from 20% to 60%.

Optionally, when the angle at each joint is in the range of 5° to 30°, metal coverage of the stent may range from 5% to 45%.

Optionally, when the angle at each joint is in the range of 30° to 90°, metal coverage of the stent may range from 3% to 15%.

Optionally, when the angle at each joint is in the range of 90° to 140°, metal coverage of the stent may range from 2% to 15%.

Optionally, each stent strut may comprise two main sections and one broadened section located between the two main sections.

Optionally, the stent mesh may comprise 8 to 24 stent struts.

Optionally, at least one of the broadened sections may comprise a cavity.

Optionally, the broadened section may comprise 1 to 10 cavities.

Optionally, the cavity may comprise a longitudinal cross-sectional shape composed of at least one of an arcuate shape, a quadrilateral shape and a triangular shape.

Optionally, the cavity may comprise a transverse cross-sectional shape composed of at least one of a circular shape, an elongate shape, a polygonal shape, a corrugated shape, an annular shape and an irregular shape.

Optionally, the cavity may be configured for a drug or radiopaque agent to be filled therein.

Optionally, when the angle at the each joint is minimized, in adjacent stent struts connected at the same one of the joints, the broadened section of one of the stent struts may not overlap with the main section of the other one of the stent struts.

Optionally, in at least one stent strut, the broadened section may have margins of the same or different widths beyond the main section at opposite sides of the stent strut along a lengthwise direction thereof.

Optionally, in at least one stent strut, the broadened section may be flush with the main section at one side of the stent strut along a lengthwise direction thereof.

Optionally, in an expanded configuration of the stent, adjacent stent struts connected at the same joint may form a V-shaped structure, wherein the sides of the adjacent stent struts, at which the main sections are flush with the broadened sections, are simultaneously located at inner side or outer side of the V-shaped structure.

Optionally, the stent may comprise at least two stent meshes that are axially connected.

Optionally, the stent may comprise at least one linking strut, and wherein the joints in adjacent stent meshes are connected through the linking strut.

Optionally, the linking struts may comprise a shape composed of at least one of a linear shape, a corrugated shape, a serrated shape, a circular shape, an annular shape, a "Ω"-like shape and an "S"-like shape.

A second aspect of the present invention provides a drug-loaded stent comprising the stent according to the first aspect of the present invention, the at least one of the broadened sections comprises a cavity configured for a drug to be filled therein.

The stent provided herein includes at least one stent mesh each including a plurality of stent struts sequentially connected circumferentially around the stent mesh, the plurality of the stent struts are sequentially connected end to end, and a joint is formed at the connected ends of adjacent stent struts. The stent mesh is configured to expand or collapse as a result of widening or narrowing of angles at the joints. Each stent strut includes main sections and broadened sections alternately arranged with the main sections. The main sections have a width smaller than a width of the broadened sections. In each stent mesh, the broadened sections of adjacent stent struts are staggered along an axis of the stent mesh. The drug-loaded stent provided herein is of a similar structure. The different widths of the broadened sections and the main sections, as well as the staggered arrangement of the broadened sections, impart to the present invention at least one of the following advantages over the prior art:
1. Reasonable space utilization and a good tradeoff between metal coverage and radial strength are achieved, overcoming the problem of failing to provide both good metal coverage and desirable radial strength associated with conventional stents.
2. The staggered arrangement of the broadened sections allows the stent to have a compact size when in a collapsed configuration, which facilitates passage of the stent through a lesion site.
3. Since the broadened sections have a relatively large surface area, cavities can be formed therein, in which a drug or radiopaque agent can be filled to increase the stent's drug loading capacity or radiopacity.

### BRIEF DESCRIPTION OF THE DRAWINGS

Those of ordinary skill in the art would appreciate that the accompanying drawings are provided to facilitate a better understanding of the present invention and do not limit the scope thereof, in which:
Fig. 1a is a schematic partial view of a stent mesh according to an embodiment of the present invention;
Fig. 1b is a schematic diagram showing the stent mesh of Fig. 1a in a collapsed configuration;
Fig. 2 is a schematic partial view of a stent mesh according to another embodiment of the present invention;
Fig. 3a is a schematic partial view of a stent mesh according to yet another embodiment of the present invention;
Fig. 3b is a schematic diagram showing the stent mesh of Fig. 3a in a collapsed configuration;
Fig. 4a is a schematic diagram showing a broadened section according to an embodiment of the present invention;
Fig. 4b is a schematic diagram showing a broadened section according to another embodiment of the present invention;
Fig. 4c is a schematic diagram showing a broadened section according to yet another embodiment of the present invention;
Fig. 5a schematically illustrates a first optional implementation of a cavity pattern according to the present invention;
Fig. 5b schematically illustrates a second optional implementation of a cavity pattern according to the present invention;
Fig. 5c schematically illustrates a third optional implementation of a cavity pattern according to the present invention;
Fig. 5d schematically illustrates a fourth optional implementation of a cavity pattern according to the present invention;
Fig. 5e schematically illustrates a fifth optional implementation of a cavity pattern according to the present invention;
Fig. 6 is a schematic partial view of a stent according to an embodiment of the present invention;
Fig. 7a is a schematic partial view of a stent mesh according to the present invention, particularly showing an implementation of joints therein;
Fig. 7b is a schematic diagram showing the stent mesh of Fig. 7a in a collapsed configuration;
Fig. 8a is a schematic partial view of a stent mesh according to the present invention, particularly showing another implementation of joints therein;
Fig. 8b is a schematic diagram showing the stent mesh of Fig. 8a in a collapsed configuration;
Fig. 9a is a schematic partial view of a stent mesh according to the present invention, particularly showing yet another implementation of joints therein; and
Fig. 9b is a schematic diagram showing the stent mesh of Fig. 9a in a collapsed configuration.

In figures,
100, a stent mesh; 110, a stent strut; 120, a joint; 130, a linking strut; 101, a main section; 102, a broadened section; 103, a main segment; 104, a transition segment; 105, a cavity; 121, a bend; and 200, a widthwise direction.

### DETAILED DESCRIPTION

Objects, advantages and features of the present invention will become more apparent upon reading the following more detailed description of the present invention with reference to the accompanying drawings. Note that the figures are provided in a very simplified form not necessarily drawn to exact scale and for the only purpose of facilitating easy and clear description of the embodiments. In addition, the structures shown in the figures are usually part of actual structures. In particular, as the figures tend to have distinct emphases, they are often drawn to different scales.

As used herein, the singular forms "a", "an" and "the" include plural referents. Herein, the term "or" is generally employed in the sense of "and/or", "several" is generally employed in the sense of "at least one", and "at least two" is generally employed in the sense of "two or more". Additionally, the use of the terms "first", "second" and "third" herein is intended for illustration only and is not to be construed as denoting or implying relative importance or as implicitly indicating the numerical number of the referenced item. Accordingly, defining an item with "first", "second" or "third" is an explicit or implicit indication of the presence of one or at least two of the items. As used herein, the term "proximal end" generally refers to an end closer to an operator, and the term "distal end" generally refers to an end closer to a patient. The terms "one end" and "the other end", as well as "proximal end" and "distal end", are generally used to refer to opposite end portions including the opposite endpoints, rather than only to the endpoints. The terms "mounting", "coupling" and "connection" should be interpreted in a broad sense. For example, a connection may be a permanent, detachable or integral connection, a mechanical or electrical connection, a direct or indirect connection with one or more intervening media, or an internal communication or interaction between two elements. As used herein, when an element is referred to as being "disposed on" another element, this is generally intended to only mean that there is a connection, coupling, engagement or transmission relationship between the two elements, which may be either direct or indirect with one or more intervening elements, and should not be interpreted as indicating or implying a particular spatial position relationship between the two elements, i.e., the element may be located inside, outside, above, under, beside, or at any other location with respect to the other element, unless the context clearly dictates otherwise. Those of ordinary skill in the art can understand the specific meanings of the above-mentioned terms herein, depending on their context.

In principle, the present invention seeks to provide a stent, which can overcome the problem of failing to provide both good metal coverage and desirable radial strength associated with conventional stents.

The invention will be described below with reference to the accompanying drawings.

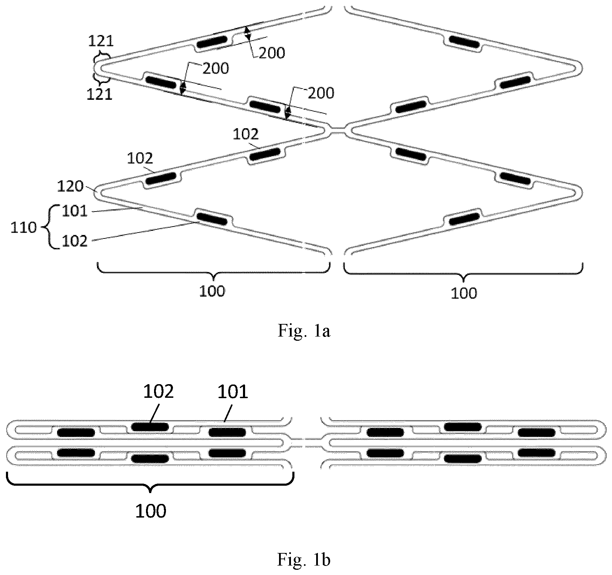

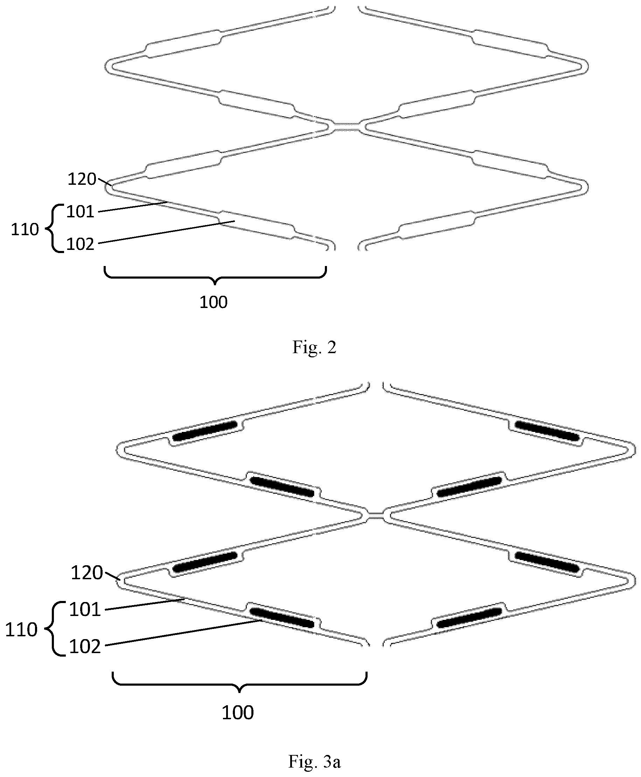

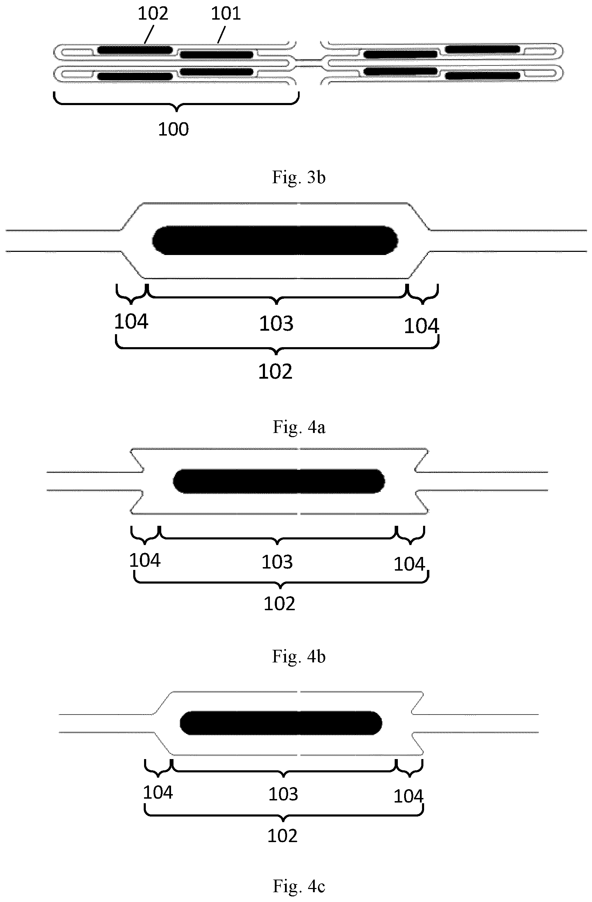

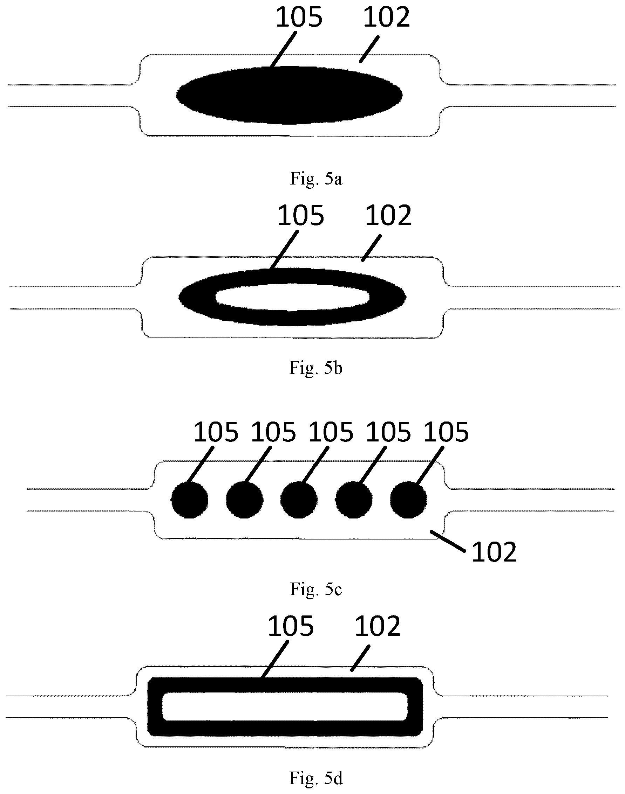

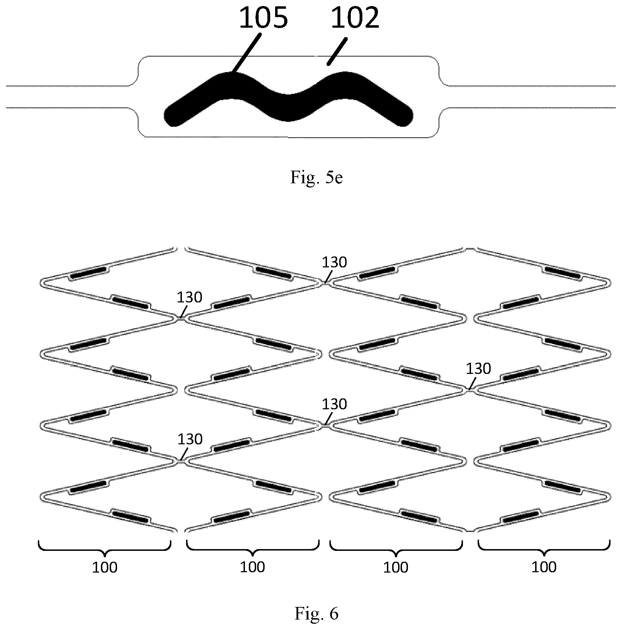

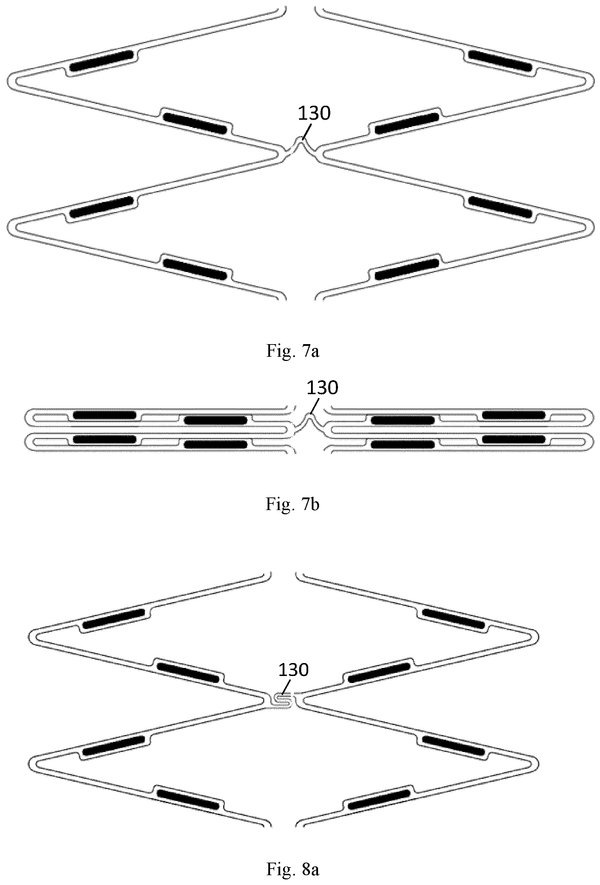

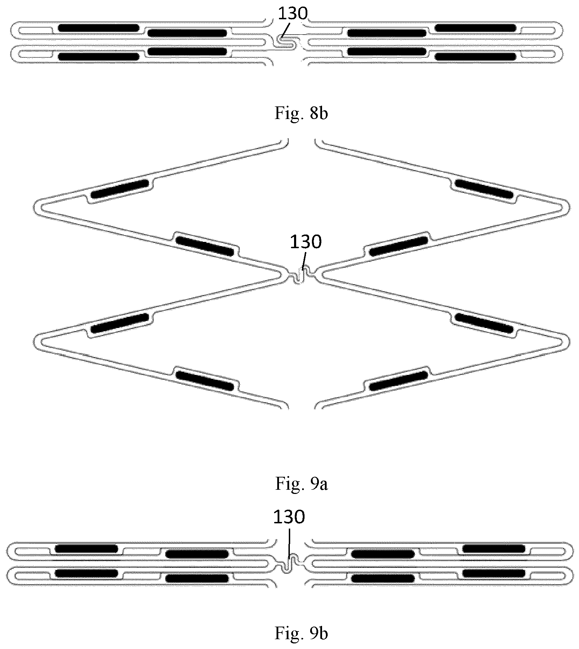

Reference will be made to Figs. 1a to 9b. Fig. 1a is a schematic partial view of a stent mesh according to an embodiment of the present invention. Fig. 1b is a schematic diagram showing the stent mesh of Fig. 1a in a collapsed configuration. Fig. 2 is a schematic partial view of a stent mesh according to another embodiment of the present invention. Fig. 3a is a schematic partial view of a stent mesh according to yet another embodiment of the present invention. Fig. 3b is a schematic diagram showing the stent mesh of Fig. 3a in a collapsed configuration. Fig. 4a is a schematic diagram showing a broadened section according to an embodiment of the present invention. Fig. 4b is a schematic diagram showing a broadened section according to another embodiment of the present invention. Fig. 4c is a schematic diagram showing a broadened section according to yet another embodiment of the present invention. Fig. 5a schematically illustrates a first optional implementation of a cavity pattern according to the present invention. Fig. 5b schematically illustrates a second optional implementation of a cavity pattern according to the present invention. Fig. 5c schematically illustrates a third optional implementation of a cavity pattern according to the present invention. Fig. 5d schematically illustrates a fourth optional implementation of a cavity pattern according to the present invention. Fig. 5e schematically illustrates a fifth optional implementation of a cavity pattern according to the present invention. Fig. 6 is a schematic partial view of a stent according to an embodiment of the present invention. Fig. 7a is a schematic partial view of a stent mesh according to the present invention, particularly showing an implementation of joints therein. Fig. 7b is a schematic diagram showing the stent mesh of Fig. 7a in a collapsed configuration. Fig. 8a is a schematic partial view of a stent mesh according to the present invention, particularly showing another implementation of joints therein. Fig. 8b is a schematic diagram showing the stent mesh of Fig. 8a in a collapsed configuration. Fig. 9a is a schematic partial view of a stent mesh according to the present invention, particularly showing yet another implementation of joints therein. Fig. 9b is a schematic diagram showing the stent mesh of Fig. 9a in a collapsed configuration.

As shown in Figs. 1a and 1b, in an embodiment, a stent is provided, which includes a plurality of stent meshes 100 each including a plurality of stent struts 110, which are connected circumferentially around the stent mesh 100, the plurality of the stent struts are sequentially connected end to end, and a joint is formed at the connected ends of adjacent stent struts. The stent meshes 100 are configured to expand or collapse as a result of widening or narrowing of angles at the joints 120. Each stent strut 110 includes at least one main section 101 and at least one broadened section 102. The main section 101 has a width smaller than a width of the broadened section 102. The broadened sections 102 of adjacent stent struts 110 are staggered along an axis of the stent mesh 100. When the angles at the joints are in a range of 0° to 5°, metal coverage of the single stent mesh ranges from 30% to 99 %. In this embodiment, the lengths are measured along an axis of the stent strut. In this embodiment, the widths are measured in directions perpendicular to axes of the respective features being measured and both tangential to an outer circumferential surface of the stent mesh 100. In Figs. 1a to 9b, the directions in which the widths are measured are perpendicular to both the axes of the features being measured and a line of sight of the viewer. In Fig. 1a, width directions are illustrated and labeled as 200. Any other width or length as mentioned herein below is measured in a similar way.

Metal coverage of the stent refers to a ratio of its total metal outer surface area to a total area of a vessel segment covered by the implanted stent. Specifically, it can be expressed as the formula: Metal Coverage = Ss/πDL × 100%, where Ss represents the metal outer surface area of the stent, D is an outer diameter of the covered segment, and L is a length of the covered segment. It would be appreciated that the metal coverage depends on the structure of the stent and thus can be increased by appropriately modifying the structure of the stent, optionally in an expanded or collapsed configuration.

It would be appreciated that, in the stent shown in Figs. 1a and 1b, there are a plurality of stent meshes 100. What is shown in Figs. 1a and 1b is only part of the stent mesh 100 (as is the case of Figs. 3a to 3b and Figs. 6 to 9b described below), and the stent mesh 100 may be constructed from multiple replicas of the portion shown in Figs. 1a and 1b, which may be connected together. In various embodiments, each single stent mesh 100 may include 8 to 24, preferably, 12 to 18, stent struts 110. Likewise, the stent includes a plurality of (e.g., 2 to 18) stent meshes 100. Each stent mesh 100 is a ringshaped structure made up of stent struts 110 which are connected end to end, and multiple such stent meshes 100 are connected axially to form the stent. When in an expanded configuration, the stent meshes 100 is as shown in Fig. 1a, the stent can be used to support human tissue such as a blood vessel. When in a collapsed configuration as shown in Fig. 1b, the stent meshes 100 are suitable for passage through a limited space, such as a lumen of a blood vessel. The main sections 101 of the stent struts 110 allow the stent to overall occupy a smaller space, while the broadened sections 102 can ensure that metal coverage of the stent is large enough to enable desirable coverage of a plaque by the stent. The different widths of the broadened sections 102 and the main sections 101, as well as the staggered arrangement of the broadened sections 102, enable the stent to have a desirable radial strength which can ensure that the stent is able to support a stenotic site while not causing damage to a blood vessel.

Referring to Fig. 2, in some embodiments, the stent provided herein may not have cavities. However, this is undesirable according to an analysis of drug-loaded stents performed by the inventors. Most existing drug loading techniques based on a self-expanding stent involve coating a surface of the stent with a drug layer or with drug-containing film. There is a known self-expanding stent, which is surface-coated with a polymer film formed by spraying drug-containing polymer nanofibers onto the stent surface. There is another known self-expanding luminal stent which is entirely or partially coated with a film attached thereto with a drug. There is yet another known stent consisting of a shape memory metal mesh with a coating film and a drug layer applied onto the film. All these techniques inevitably suffer from premature wear or peeling of the drug layer during crimping, loading and delivery of the self-expanding stent, and filling the drug into cavities formed in the stent is considered as a good solution to this problem. In fact, this design has found use in some stents for treating stenosis of extracranial arteries, such as coronary drug-eluting stents. Therefore, in some embodiments, cavities for containing a drug therein may be formed in the broadened sections, in order to increase drug loading capacity of the metal sections. The drug loading capacity can be achieved because: 1) the broadened sections 102 are wider and therefore satisfy the basic space requirements for drug loading; and 2) loading the drug in the cavity instead of coating it onto the stent surface circumvents the problem of premature wear or peeling of the drug during crimping, loading and delivery of the stent. This arrangement enables desirable space utilization and takes into account of both drug loading capacity and radial strength, allowing the stent to have a compact size in the collapsed configuration, which facilitates passage of the stent through a lesion site. Therefore, the stent can provide satisfactory ability to pass through a lesion site, desirable drug loading capacity and good radial strength.

With the above arrangement, the stent can have a radial dimension smaller than or equal to 0.0419 cm (0.0165 inches) when in the collapsed configuration, which enables the stent to be used with the smallest available delivery microcatheters. In all embodiments, the stent may have an outer diameter of 0.3 to 0.7 mm when in the collapsed configuration, which allows the stent to be used with microcatheters with an inner diameter in the range of 0.033 cm (0.013 inches) to 0.068 cm (0.027 inches). In all embodiments, the stent struts may have a wall thickness of 0.05 mm to 0.07 mm.

The inventors have carried out tests with various ratios of total length of the broadened section(s) 102 to the length of the stent strut 110. All the tested stents had an outer diameter of 0.53 mm in the collapsed configuration, a total length of 9.68 mm in the collapsed configuration, a stent strut thickness of 0.07 mm and a stent mesh count of 6. Starting with all these same initial dimensions in the collapsed configuration, the stents expanded to a radial dimension of 3 mm, when their radial strength and metal coverage were measured. The results are shown in Table 1.

**Table 1 Dependence of Radial Strength and Metal Coverage on Ratio of Broadened Sections**

| No. | ratio of total length of broadened section(s) to length of stent strut (%) | ratio of total length of broadened sections in adjacent stent struts to length of stent strut (%) | radial strength (kPa) | metal coverage (%) |
|---|---|---|---|---|
| 1 | 0 | 0 | 6.77 | 7.51 |
| 2 | 0 | 100 | 10.5 | 13.12 |
| 3 | 50 | 50 | 9 | 11.31 |
| 4 | 10 | 90 | 9.1 | 12.85 |
| 5 | 5 | 5 | 6.92 | 8.62 |
| 6 | 5 | 95 | 9.7 | 12.93 |
| 7 | 30 | 30 | 8.71 | 11.11 |
| 8 | 15 | 15 | 7.23 | 8.93 |
| 9 | 20 | 20 | 7.56 | 9.24 |
| 10 | 25 | 75 | 9.23 | 12.84 |

As can be seen from the data in Table 1, in comparison to the results of Test No. 1 serving as a control test, the stents with ratios of total length of the broadened section(s) 102 in one stent strut to length of the stent strut 110 ranging from 5% to 95% exhibit both desirable radial strength and sufficiently large metal coverage.

As can be also seen from the data in Table 1, the ratio of total length of the broadened section(s) 102 in one stent strut to length of the stent strut 110 is preferably 15% to 75%, more preferably 20% to 50%. In some particular embodiments, the ratio of total length of the broadened section(s) 102 in one stent strut to length of the stent strut 110 may be 5%, 10%, 15%, 20%, 25%, 30%, 35%, 40%, 45%, 50%, 55%, 60%, 65%, 70%, 75%, 80%, 85%, 90%, 95% or the like.

Additionally, the ratio of the total length of the broadened section(s) 102 in any adjacent two stent struts 110 to the length of the stent strut 110 does not exceed 100%. As also can be seen from the data in Table 1, a ratio of the total length of the broadened section(s) 102 in any adjacent stent struts 110 to the length of stent strut 110 may be the same or different.

As also can be seen from the data in Table 1, the stents show radial strength of 1 kPa to 100 kPa.

The inventors also tested stents of various structures on their radial strength at a diameter of 2 mm to 5 mm, radial strength at minimized angles at the joints and metal coverage. Among the tested samples, Experimental Samples 1 to 3 were stents with alternating main section 101 and broadened section 102, and Comparative Samples 1 to 4 are stents each with a constant strut width. The results are shown in Table 2.

**Table 2 Radial Strength and Metal Coverage of Samples**

| Sample | Radial Strength at Diameter of 2 mm to 5 mm (kPa) | Radial Strength at Minimized Angles at Joints (kPa) | Metal Coverage (%) |
|---|---|---|---|
| Experimental Sample 1 | 5 to 9 | 128 | 10.4 |
| Experimental Sample 2 | 8 to 12 | 145 | 10.8 |
| Experimental Sample 3 | 10 to 11 | 166 | 11.3 |
| Comparative Sample 1 | 8 to 13 | 263 | 10.8 |
| Comparative Sample 2 | 4 to 9 | 160 | 10 |
| Comparative Sample 3 | 1 to 4 | 75 | 5 |
| Comparative Sample 4 | 2 to 5 | 103 | 6.5 |

As can be seen from the data in Table 2, the stent samples according to the present embodiment exhibited radial strength of 1 kPa to 300 kPa.

As can be also seen from the data in Table 2, when the angles at the joints were minimized, the stent samples showed radial strength in the range of 50 kPa to 300 kPa. More preferably, when the angles at the joints are minimized, the radial strength of the stent ranges from 100 kPa to 200 kPa. With radial strength in this range and the angles being minimized at the joints, the stent will have great metal coverage while showing desirable radial strength when expanded to a deployed configuration (e.g., with a diameter of 2 mm to 5 mm). Suitable radial strength in the deployed configuration allows the stent to provide desirable support at a target site in a blood vessel while not causing damage to the vulnerable vessel wall. The angles at the joints are minimized when the stent is in the collapsed configuration for delivery in a blood vessel during clinical use. In the collapsed configuration, the stent is also desired to have appropriate radial strength because excessive radial strength in the collapsed configuration may make the stent difficult to deliver through the blood vessel to a target site, or cause other problems. It would be appreciated that the radial strength of the stent depends on its structure, and suitable radial strength can be obtained only when the structure of the stent is properly designed.

As can be also seen from the data in Table 2, when expended to a diameter of 2.0 mm to 5.0 mm, the stent samples showed radial strength in the range of 1 kPa to 30 kPa. In this diameter range of 2.0 mm to 5.0 mm, the radial strength is more preferably in the range of 1 kPa to 15 kPa, even more preferably in the range of 1 kPa to 12 kPa.

In various embodiments, the length of the stent strut 110 may range from 1 mm to 2.5 mm. In one preferred embodiment, the length of the stent strut 110 may range from 1.2 mm to 1.5 mm. The stent strut 110 may be generally linear in shape, or may have one or more curved portions, but they extend overall in the lengthwise direction.

In various embodiments, the stent strut 110 may have a width of 20 mm to 150 mm. In various embodiments, the width of the main section 101 may range from 30 µm to 50 µm. In various embodiments, the width of the broadened section 102 may range from 90 µm to 120 µm.

The metal coverage of the stent is determined by its structure and the angles at the joints 120. When the structure of the stent is uniform, the angles at the joints 120 are defined by the adjacent two stent struts 110 connected at joint 120. When the angles at the joints 120 are 0°, the stent is the aforementioned collapsed configuration where the two stent struts 110 connected at any joint 120 define an angle of 0° therebetween, resulting in large metal coverage of the stent. When the angles at the joints 120 are 30° to 120°, the stent is the aforementioned expanded configuration where it shows reduced metal coverage. In the collapsed configuration, at a given total metal surface area of the stent and a given thickness of the struts, larger metal coverage means a smaller outer diameter of the stent, which allows the stent to be more easily advanced in a blood vessel and to be delivered to a more distant target site or a target site in a narrower blood vessel. In the expanded configuration, larger metal coverage allows the stent to more desirably cover a plaque at a lesion site, providing better therapeutic efficacy. The design with alternating wide and narrow strut sections enables the stent to have effectively improved metal coverage while exhibiting satisfactory radial strength. Metal coverage of stent meshes 100 with different ratios of broadened sections at various angles is shown in Table 3.

**Table 3 Metal Coverage of Stent Meshes with Different Ratios of Broadened Sections at Various Angles**

| No. | ratio of total length of broadened section(s) to length of stent strut (%) | ratio of total length of broadened sections in adjacent stent struts to length of stent strut (%) | metal coverage of single stent mesh (%) | | | | | |
|---|---|---|---|---|---|---|---|---|
| | | | angle at 0° | angle at 5° | angle at 30° | angle at 60° | angle at 90° | angle at 120° |
| 1 | 5 | 5 | 38.44 | 33.86 | 8.81 | 4.78 | 4.16 | 4.03 |
| 2 | 30 | 30 | 53.50 | 47.12 | 11.18 | 6.65 | 5.79 | 5.61 |
| 3 | 50 | 50 | 94.39 | 83.14 | 11.88 | 11.74 | 10.21 | 9.89 |
| 4 | 10 | 90 | 96.45 | 84.95 | 12.02 | 11.99 | 10.43 | 10.11 |
| 5 | 5 | 95 | 96.14 | 84.67 | 12.06 | 11.96 | 10.40 | 10.08 |
| 6 | 0 | 100 | 95.82 | 84.39 | 12.89 | 11.92 | 10.37 | 10.04 |

As can be seen from the data in Table 3, when the stent is in a configuration where the angles at the joints in the stent range from 0° to 5°, the stent mesh 100 has metal coverage of 30% to 99%. As can be also seen from Table 3, in said configuration of the stent where the angles at the joints in the stent range from 0° to 5°, the stent mesh 100 is preferred to have metal coverage of 80% to 99%.

As can be seen from the data in Table 3, the angles at the joints 120 may range from 0° to 120°. In some other embodiments, the angles at the joints 120 may range from 0° to 140°. When the angles at the joints 120 are 0° or almost 0°, the stent is in the collapsed configuration. This collapsed configuration can be achieved by crimping the stent into a guide sheath before loading into a microcatheter. The stent remains in the collapsed configuration after it is loaded into the microcatheter and during the subsequent delivery therein. Notably, it is not the case that the stent is in the collapsed configuration only when the angles at the joints 120 are 0°. Depending on an inner diameter of the microcatheter, the stent may be loaded therein with various angles at the joints 120. A larger inner diameter of the microcatheter will lead to larger angles at the joints 120. For example, the angles at the joints 120 may be 0° to 5° in the collapsed configuration of the stent. When the angles at the joints 120 lie between 30° and 120°, the stent comprises the expanded configuration. In some embodiments, when the stent expands to an outer diameter of 2 mm to 3 mm, the angles at the joints 120 are in the range of 30° to 90°. It is noted that the expanded configuration is not necessarily a nature state of the stent where it fully expands. Rather, it may only expand to a predetermined outer diameter in the expanded configuration. For example, when placed in a tube with an inner diameter of 2 mm to 3 mm, the stent may expand to an outer diameter of 2 mm to 3 mm in the expanded configuration. At this time, the angles at the joints 120 are greater than the angles in the collapsed configuration. After being taken out of the tube or transferred into a tube with a greater inner diameter, the stent may further expand, leading to an increase in the angles at the joints 120.

When the angles at the joints are in the range of 5° to 30°, the single stent mesh shows metal coverage of 5% to 90%.

As can be seen from the data in Table 3, when the angles at the joints are in the range of 30° to 90°, the single stent mesh shows metal coverage of 4% to 15%. As can be also seen from the data in Table 3, the metal coverage of the single stent mesh 100 is preferred to be 8% to 15% when the angles at the joints in the stent is in the range of 30° to 90°.

When the angles at the joints are in the range of 90° to 140°, the single stent mesh shows metal coverage of 3% to 12%.

Apart from the metal coverage of the individual stent meshes 100, overall metal coverage of the stent may also depend on linking struts 130 between the stent meshes. The longer the linking struts 130, the more distant the stent meshes 100 are spaced apart from each other. Due to the presence of the linking struts 130, the overall metal coverage of the stent is smaller than the metal coverage of the individual stent meshes 100. When the linking struts 130 are equally long and of the same structure, the greater the number of linking struts between the stent meshes 100, the greater the metal coverage of the stent. In addition, the metal coverage of the stent may also depend on the structure of the linking struts. In the embodiments provided herein, when the angles at the joints are in the range of 0° to 5°, the overall metal coverage of the stent is 20% to 60%, preferably 35 to 60%; when the angles at the joints are in the range of 5° to 30°, the overall metal coverage of the stent is 5% to 45%, preferably 8% to 40%; when the angles at the joints are in the range of 30° to 90°, the overall metal coverage of the stent is 3% to 15%, preferably 8% to 15%; and when the angles at the joints are in the range of 90° to 140°, the overall metal coverage of the stent is 2% to 15%, preferably 5% to 15%.

In various embodiments, the stent strut 110 may include 1 to 10 broadened sections 102. Preferably, the stent strut 110 includes one broadened section 102. This enables good space utilization and ensures large metal coverage and/or a sufficient drug load. Moreover, the stent strut 110 may include 1 to 11 main sections 101. Preferably, the stent strut 110 includes 2 main sections 101. In the same stent, the main sections 101 are alternately arranged with the broadened section(s) 102. A ratio of width of the main sections 101 to the width of the broadened sections 102 may range from 1/4 to 2/3. Preferably, the width ratio is 1/3, in order for good space utilization to be achieved. Adjacent stent struts 110 may include the same or different numbers of broadened sections 102. Each stent strut 110 may include one or more broadened sections 102. Referring to Fig. 1a, in one embodiment, the stent strut 110 includes three main sections 101 and two broadened sections 102 alternating with the broadened sections 102. Each broadened section is located between two main sections. Moreover, in the same stent mesh 100, an adjacent stent strut includes two main sections 101 and one broadened section 102 located between the main sections. With continued reference to Fig. 2, in a preferred embodiment, the stent strut 110 in a stent mesh 100 includes two main sections 101 and one broadened section 102 located between the main sections.

The stent mesh 100 further includes bends 121 for connecting the stent struts 110. In various embodiments, a ratio of width of the main sections 101 to the bends 121 may range from 1/2 to 3/4. Preferably, the ratio is 3/5, which can prevent breakage at the joints 120 while enabling the stent to have low strength.

In various embodiments, a ratio of length of the stent strut 110 to length of the shortest main section 101 therein may be greater than or equal to 10/3, preferably 6/1.

In various embodiments, a ratio of length of the stent strut 110 to length of the bend 121 may be greater than or equal to 8. Preferably, this ratio is 12, which can prevent breakage at the joints 120 while enabling the stent to have low strength.

Preferably, when the angles at the joints 120 are minimized, the broadened section 102 in one stent strut 110 does not overlap with the main section 101 in an adjacent stent strut 110 connected at the same joint 120. During design, backward inference may be used to calculate a dimension of the broadened sections 102 from a radial distance between the main sections 101 in adjacent stent struts 110 at the minimized angles, which allow the stent to have designed overall structural strength and a designed service lifespan. This can additionally enhance space utilization and enable the stent to have an even smaller overall size. In some embodiments, the main sections 101 in different pairs of adjacent stent struts 110 connected at respective joints 120 may be spaced by different radial distances when the angles are minimized. In this case, the widths of the broadened sections 102 in the stent struts 110 may be individually designed in order to achieve good space utilization. Alternatively, all the broadened sections 102 may be designed with the same width, according to the smallest one of the radial distances of all the pairs of adjacent stent struts 110 at the minimized angles. This represents a low-cost design approach.

It would be appreciated that the above-discussed embodiments are preferred, but in some special cases, when the angles at the joints 120 are minimized, in adjacent stent struts 110 connected at the same joint 120, the radial distance between their main sections 101 may also be smaller than the widths of protrusion of the broadened sections 102 relative to the main sections 101 on one side. This can also achieve good space utilization.

As shown in Fig. 2, in one embodiment, the broadened sections 102 have margins of the same width beyond the main sections 101 at opposite sides of the stent struts along the lengthwise direction thereof. Center axes of the broadened sections 102 coincide with center axes of the stent struts 110, optionally with offsets within a manufacturing tolerance range.

In some other embodiments, the broadened sections 102 may have margins of different widths beyond the main sections 101 at opposite sides of the stent struts along the lengthwise direction thereof. For example, as shown in Figs. 3a and 3b, in one embodiment, the broadened sections 102 are flush with the main sections 101 at one side of the stent strut along the lengthwise direction thereof. Preferably, in the expanded configuration of the stent, the adjacent stent struts 110 connected at the same joint 120 forms a V-shaped structure, wherein the sides of the adjacent stent struts 110, at which the broadened sections 102 are flush with the main sections 101, are both located at inner or outer sides of the V-shaped structure.

In other embodiments, the sides of the adjacent stent struts 110, at which the broadened sections 102 are flush with the main sections 101, may be located at sides of the V-shaped structure facing the same direction. In other words, in the expanded configuration of the stent, the adjacent stent struts 110 connected at the same joint 120 forms a V-shaped structure, wherein the sides of the adjacent stent struts 110, at which the broadened sections 102 are flush with the main sections 101, are not both located at inner or outer sides of the V-shaped structure. That is, one of the sides of the adjacent stent struts 110, at which the broadened sections 102 are flush with the main sections 101, is located at the inner side of the V-shaped structure, while the other of the sides of the adjacent stent struts 110, at which the broadened sections 102 are flush with the main sections 101, is located at the outer side of the V-shaped structure.

Referring to Figs. 4a to 4c, each broadened section 102 includes a main segment 103 and transition segments 104 each joining the main segment 103 to one main section 101. The transition segments 104 may comprise any of various shapes. For example, in one embodiment, the transition segments 104 are each tapered from the main segment 103 toward the main section 101 (see Fig. 4a). In an alternative embodiment, the transition segments 104 each include two outwardly open transition notches on opposite sides of the main section 101. The transition segments 104 are as wide as the main segment 103, and the openings of the transition notches are tapered from the main section 101 toward the main segment 103 (see Fig. 4b). In another alternative embodiment, out of the transition segments 104 of all the broadened sections 102, at least some comprise at least one of the above-discussed shapes (i.e., any of the examples shown in Figs. 4a, 4b and 4c). It would be appreciated that the transition segments 104 may also be configured otherwise. In some other embodiments, for example, in the embodiment shown in Fig. 3a, the broadened section 102 may only include the main segment 103 and, in this case, the main segment 103 is directly joined to the main sections 101.

Since the broadened sections have a relatively large surface area, cavities can be formed therein, in which a drug or radiopaque agent may be filled to increase the stent's drug loading capacity or radiopacity. Referring to Figs. 5a to 5e, the broadened sections 102 may include cavities 105, the cavity is formed by cavity pattern(s), the cavity pattern comprises at least one of a solid pattern, a hollow pattern, a continuous pattern and a discontinuous pattern. Each cavity may have a shape comprising at least one of a cylinder, a cuboid and a triangular prism. Each cavity may have a transverse cross-sectional shape comprising at least one of a circular shape, an elongate shape, a polygonal shape, a corrugated shape, an annular shape and an irregular shape. Each cavity may have a longitudinal cross-sectional shape comprising at least one of an arcuate shape, a quadrilateral shape and a triangular shape. Notably, the transverse cross-sectional shape refers to the shape of a cross-section taken parallel to an outer surface of the broadened section 102 in which the cavity is formed, and the longitudinal cross-sectional shape refers to the shape of a cross-section taken perpendicular to both the outer surface and a lengthwise direction of the broadened section 102. The cavities 105 are configured for a drug or radiopaque agent to be filled therein. In some embodiments, the cavities 105 may be through slots extending through the stent strut 110 across its entire thickness. In some other embodiments, the cavities 105 may be blind holes extending through a partial thickness of the stent struts 110. It would be appreciated that Figs. 4a to 4c are meant to particularly exemplify and illustrate the transition segments 104, and Figs. 5a to 5e are meant to particularly exemplify and illustrate the cavities 105. It is in no way intended that when the transition segments 104 are configured as shown in any of Figs. 4a to 4c, the cavities 105 have to be shaped as shown in the same figure, or that when the cavities are configured as shown in any of Figs. 5a to 5e, the transition segments 104 have to be shaped as shown in the same figure. These implementations may be combined in any combination.

In various embodiments, a ratio of a maximum width of the cavity pattern to the width of the broadened sections 102 may be in the range of 1/3 to 4/5. Preferably, this ratio is 1/2, because it can enable good space utilization and ensure a sufficient drug load. The width of the cavity pattern is measured in the same direction as the width of the broadened sections 102 is measured.

Referring to Fig. 1a, in one embodiment, the stent includes at least two stent meshes 100 that are axially connected. The stent may include 4 to 18 stent meshes 100, preferably 6 to 10 stent meshes 100. The stent meshes may be connected to each other in any suitable way. In a preferred embodiment, referring to Fig. 6, the stent includes linking struts 130, and the joints 120 in adjacent stent meshes 100 are aligned and connected by the linking struts 130. In the embodiment shown in Fig. 6, the joints 120 in adjacent stent meshes 100 are aligned, and adjacent stent meshes 100 are in mirrored symmetry with each other. Additionally, referring to Fig. 6, the linking struts 130 may connect pairs of joints 120 interposed between non-connected pairs circumferentially along the stent. This arrangement can facilitate the widening or narrowing of the angles at the joints and enhance space utilization. It would be appreciated that, in alternative embodiments, the joints 120 may be not aligned and the linking struts may be not at the joints 120. Moreover, when the joints 120 are aligned and connected by the linking struts 130, the connections may be made in another way than as discussed above. For example, all pairs of aligned joints 120 may be each connected with a linking strut 130. Alternatively, the pairs of aligned joints 120 may be connected with the linking struts 130 at an interval of two non-connected pairs circumferentially along the stent. Still other arrangements are possible.

In various embodiments, a ratio of width of the main sections 101 to width of the linking struts 130 may be in the range of 1/2 to 1. This ratio is preferred to be 1/2, in order to satisfactorily prevent breakage of the linking struts 130.

Referring to Figs. 1a to 1b and 7a to 9b, the linking struts 130 may have a shape comprising at least one of a linear shape, a corrugated shape, a serrated shape, a circular shape, an annular shape, a "Ω"-like shape and an "S"-like shape. The linking struts 130 are preferred to be non-linear in shape, for example, corrugated, "Ω"-shaped or "S"-shaped, because these shapes can facilitate overall collapse and expansion of the stent. It would be appreciated that various implementations of the linking struts 130 may be combined with those of the above-discussed cavities 105, transition segments 104 and broadened sections 102 in any combination.

In other embodiments, cavities may also be formed in the linking struts 130, though this is not favorable from the point of view of overall structural reliability.

Referring to Figs. 1 to 3b and 7a to 9b, in some embodiments, when the angles at the joints 120 are minimized, in any one of stent mesh 100, the broadened sections 102 in adjacent stent struts 110 may be spaced apart by certain distances along the axis of the stent mesh 100 (see Figs. 1b, 7b and 9b). In a more preferred embodiment, when the angles at the joints 120 are minimized, in each stent mesh 100, the broadened sections 102 in adjacent stent struts 110 may come into snug contact along the axis of the stent mesh 100 (see Figs. 3b and 8b).

In some embodiments, the stent may be formed of a cobalt-based alloy, a magnesium alloy, a nickel-titanium alloy, stainless steel or the like, or a combination thereof.

There is also provided herein a drug-loaded stent differing from the stent as defined above mainly in including cavities 105, in which a drug or radiopaque agent is filled.

For details of other features of the drug-loaded stent, reference can be made to the above description of this specification in connection with the stent.

This invention provides a stent including at least one stent mesh 100 each including a plurality of stent struts 110, the plurality of the stent struts 110 are sequentially connected end to end, and a joint 120 is formed at the connected ends of adjacent stent struts 110. The stent mesh 100 is configured to expand or collapse as a result of widening or narrowing of angles at the joints 120. Each stent strut 110 includes at least one main section 101 and at least one broadened section 102. The main section 101 is alternately arranged with the broadened section 102. The main section 101 has a width smaller than a width of the broadened section 102. In each stent mesh 100, the broadened sections 102 of adjacent stent struts 110 are staggered along an axis of the stent mesh 100. This invention also provides a drug-loaded stent of a similar structure. The different widths of the broadened sections 102 and the main sections 101, as well as the staggered arrangement of the broadened sections 102, enable reasonable space utilization and achieve a good tradeoff between metal coverage and radial strength. In this way, the stent is allowed to have a compact size when in a collapsed configuration, which facilitates passage of the stent through a lesion site. Thus, the problem of failing to provide both good metal coverage and desirable radial strength associated with conventional stents is overcome.

The description presented above is merely that of some preferred embodiments of the present invention and is not intended to limit the scope thereof in any sense. The invention is defined by

## Claims

1. A stent, comprising at least one stent mesh (100), wherein each stent mesh (100) comprises a plurality of stent struts (110) sequentially connected circumferentially around the stent mesh (100), wherein the plurality of the stent struts (110) are sequentially connected end to end, and a joint (120) is formed at the connected ends of adjacent stent struts (110), wherein the stent mesh (100) is configured to expand or collapse as a result of widening or narrowing of angles at the joints (120),
wherein the stent strut (110) comprises at least one main section (101) and at least one broadened section (102) alternately arranged with the at least one main section (101), wherein the main section (101) has a width smaller than a width of the broadened section (102); and wherein in each stent mesh (100), the broadened sections (102) of adjacent stent struts (110) are staggered along an axis of the stent mesh (100);
**characterised in that**, a radial strength of the stent ranges from 1 kPa to 300 kPa; and
**in that**, when the stent is in an expanded configuration with a diameter of 2.0 mm to 5.0 mm, the radial strength of the stent ranges from 1 kPa to 30 kPa.

2. The stent according to claim 1, wherein when the angle at each joint (120) is minimized, the radial strength of the stent ranges from 50 kPa to 300 kPa.

3. The stent according to claim 1, wherein when the angle at each joint (120) is minimized, the radial strength of the stent ranges from 100 kPa to 200 kPa.

4. The stent according to claim 1, wherein when the stent is in the expanded configuration with the diameter of 2.0 mm to 5.0 mm, the radial strength of the stent ranges from 1 kPa to 15 kPa, optionally wherein when the stent is in the expanded configuration with the diameter of 2.0 mm to 5.0 mm, the radial strength of the stent ranges from 1 kPa to 12 kPa.

5. The stent according to claim 1, wherein the angle at each joint (120) ranges from 0° to 140°; or
wherein each stent strut (110) comprises two main sections (101) and one broadened section (102) located between the two main sections (101); or
wherein the stent mesh (100) comprises 8 to 24 stent struts (110); or
wherein when the angle at each joint (120) is minimized, in adjacent stent struts (110) connected at a same joint (120), the broadened section (102) of one of the stent struts (110) does not overlap with the main section (101) of the other one of the stent struts (110); or
wherein in at least one stent strut (110), the broadened section (102) has margins of a same width or different widths beyond the main section (101) at opposite sides of the stent strut (110) along a lengthwise direction thereof; or
wherein in at least one stent strut (110), the broadened section (102) is flush with the main section (101) at one side of the stent strut (110) along a lengthwise direction thereof, optionally wherein in an expanded configuration of the stent, adjacent stent struts (110) connected at a same joint (120) form a V-shaped structure, wherein the sides of the adjacent stent struts (110), at which the main sections (101) are flush with the broadened sections (102), are simultaneously located at an inner side or an outer side of the V-shaped structure.

6. The stent according to claim 1, wherein when the angle at each joint (120) is in a range of 0° to 5°, a metal coverage of the stent mesh (100) ranges from 30% to 99%.

7. The stent according to claim 1, wherein when the angle at each joint (120) is in a range of 5° to 30°, a metal coverage of the stent mesh (100) ranges from 5% to 90%; or.
wherein when the angle at each joint (120) is in a range of 30° to 90°, a metal coverage of the stent mesh (100) ranges from 4% to 15%, optionally wherein when the angle at each joint (120) is in the range of 30° to 90°, the metal coverage of the stent mesh (100) ranges from 8% to 15%.

8. The stent according to claim 1, wherein when the angle at each joint (120) is in a range of 90° to 140°, a metal coverage of the stent mesh (100) ranges from 3% to 12%.

9. The stent according to claim 1, wherein when the angle at each joint (120) is in a range of 0° to 5°, a metal coverage of the stent ranges from 20% to 60%.

10. The stent according to claim 1, wherein when the angle at each joint (120) is in a range of 5° to 30°, a metal coverage of the stent ranges from 5% to 45%.

11. The stent according to claim 1, wherein when the angle at each joint (120) is in a range of 30° to 90°, a metal coverage of the stent ranges from 3% to 15%.

12. The stent according to claim 1, wherein when the angle at each joint (120) is in a range of 90° to 140°, a metal coverage of the stent ranges from 2% to 15%.

13. The stent according to claim 1, wherein at least one of the broadened sections (102) comprises a cavity, optionally wherein:
the broadened section (102) comprises 1 to 10 cavities (105); or
the cavity (105) comprises a longitudinal cross-sectional shape comprising at least one of an arcuate shape, a quadrilateral shape and a triangular shape; or
the cavity (105) comprises a transverse cross-sectional shape comprising at least one of a circular shape, an elongate shape, a polygonal shape, a corrugated shape, an annular shape and an irregular shape; or
the cavity (105) is configured for a drug or radiopaque agent to be filled therein.

14. The stent according to any one of claims 1 to 13, wherein the stent comprises at least two stent meshes (100) that are axially connected, optionally wherein the stent comprises at least one linking strut (130), and wherein the joints (120) in adjacent stent meshes (100) are connected through the linking strut (130), optionally wherein the linking strut (130) comprises a shape comprising at least one of a linear shape, a corrugated shape, a serrated shape, a circular shape, an annular shape, a "Ω"-like shape and an "S"-like shape.

15. A drug-loaded stent, comprising the stent of claim 1, wherein the at least one of the broadened sections (102) comprises a cavity (105) configured for a drug to be filled therein.

## Patentansprüche

1. Stent, der mindestens ein Stentgeflecht (100) umfasst, wobei jedes Stentgeflecht (100) eine Vielzahl von Stentstreben (110) umfasst, die nacheinander umfänglich um das Stentgeflecht (100) herum verbunden sind, wobei die Vielzahl der Stentstreben (110) nacheinander an den Enden miteinander verbunden sind, und eine Verbindungsstelle (120) an den verbundenen Enden benachbarter Stentstreben (110) gebildet ist, wobei das Stentgeflecht (100) konfiguriert ist, um sich als Ergebnis von Aufweiten oder Verengen der Winkel an den Verbindungsstellen (120) auszudehnen oder zusammenzuziehen,
wobei die Stentstrebe (110) mindestens einen Hauptabschnitt (101) und mindestens einen verbreiterten Abschnitt (102) umfasst, die abwechselnd mit dem mindestens einen Hauptabschnitt (101) angeordnet sind, wobei der Hauptabschnitt (101) eine Breite aufweist, die kleiner ist als eine Breite des verbreiterten Abschnitts (102); und wobei in jedem Stentgeflecht (100) die verbreiterten Abschnitte (102) benachbarter Stentstreben (110) entlang einer Achse des Stentnetzes (100) versetzt sind;
**dadurch gekennzeichnet, dass** eine radiale Festigkeit des Stents von 1 kPa bis 300 kPa reicht; und
dadurch, dass, wenn sich der Stent in einer ausgedehnten Konfiguration mit einem Durchmesser von 2,0 mm bis 5,0 mm befindet, die radiale Festigkeit des Stents von 1 kPa bis 30 kPa reicht.

2. Stent nach Anspruch 1, wobei, wenn der Winkel an jeder Verbindungsstelle (120) minimiert wird, die radiale Festigkeit des Stents von 50 kPa bis 300 kPa reicht.

3. Stent nach Anspruch 1, wobei, wenn der Winkel an jeder Verbindungsstelle (120) minimiert wird, die radiale Festigkeit des Stents von 100 kPa bis 200 kPa reicht.

4. Stent nach Anspruch 1, wobei wenn sich der Stent in der ausgedehnten Konfiguration mit einem Durchmesser von 2,0 mm bis 5,0 mm befindet, die radiale Festigkeit des Stents von 1 kPa bis 15 kPa reicht, wobei, wenn sich der Stent optional in der ausgedehnten Konfiguration mit dem Durchmesser von 2,0 mm bis 5,0 mm befindet, die radiale Festigkeit des Stents von 1 kPa und 12 kPa reicht.

5. Stent nach Anspruch 1, wobei der Winkel an jeder Verbindungsstelle (120) von 0° bis 140° reicht; oder
wobei jede Stentstrebe (110) zwei Hauptabschnitte (101) und einen zwischen den beiden Hauptabschnitten (101) gelegenen verbreiterten Abschnitt (102) umfasst; oder
wobei das Stentgeflecht (100) 8 bis 24 Stentstreben (110) umfasst; oder
wobei, wenn der Winkel an jeder Verbindung (120) minimiert ist, bei benachbarten Stentstreben (110), die an einer gleichen Verbindung (120) verbunden sind, der verbreiterte Abschnitt (102) einer der Stentstreben (110) sich nicht mit dem Hauptabschnitt (101) der anderen der Stentstreben (110) überlappt; oder
wobei in mindestens einer Stentstrebe (110) der verbreiterte Abschnitt (102) über den Hauptabschnitt (101) hinaus an gegenüberliegenden Seiten der Stentstrebe (110) entlang einer Längsrichtung davon Ränder mit einer gleichen oder unterschiedlichen Breite aufweist; oder
wobei in mindestens einer Stentstrebe (110) der verbreiterte Abschnitt (102) an einer Seite der Stentstrebe (110) entlang einer Längsrichtung davon mit dem Hauptabschnitt (101) bündig ist, wobei optional in einer ausgedehnten Konfiguration des Stents benachbarte Stentstreben (110), die an einer gleichen Verbindungsstelle (120) verbunden sind, eine V-förmige Struktur bilden, wobei die Seiten der benachbarten Stentstreben (110), an denen die Hauptabschnitte (101) mit den verbreiterten Abschnitten (102) bündig sind, gleichzeitig an einer Innen- oder einer Außenseite der V-förmigen Struktur gelegen sind.

6. Stent nach Anspruch 1, wobei, wenn der Winkel an jeder Verbindungsstelle (120) in einem Bereich von 0° bis 5° liegt, eine Metallbedeckung des Stentgeflechts (100) von 30% bis 99% reicht.

7. Stent nach Anspruch 1, wobei, wenn der Winkel an jeder Verbindungsstelle (120) in einem Bereich von 5° bis 30° liegt, eine Metallbedeckung des Stentgeflechts (100) von 5% bis 90% reicht; oder
wobei, wenn der Winkel an jeder Verbindungsstelle (120) in einem Bereich von 30° bis 90° liegt, eine Metallabdeckung des Stentgeflechts (100) von 4% bis 15% reicht, wobei optional, wenn der Winkel an jeder Verbindungsstelle (120) im Bereich von 30° bis 90° liegt, die Metallabdeckung des Stentgeflechts (100) von 8% bis 15% reicht.

8. Stent nach Anspruch 1, wobei, wenn der Winkel an jeder Verbindungsstelle (120) in einem Bereich von 90° bis 140° liegt, eine Metallbedeckung des Stentgeflechts (100) von 3% bis 12% reicht.

9. Stent nach Anspruch 1, wobei, wenn der Winkel an jeder Verbindungsstelle (120) in einem Bereich von 0° bis 5° liegt, eine Metallbedeckung des Stents von 20% bis 60% reicht.

10. Stent nach Anspruch 1, wobei, wenn der Winkel an jeder Verbindungsstelle (120) in einem Bereich von 5° bis 30° liegt, eine Metallbedeckung des Stents von 5% bis 45% reicht.

11. Stent nach Anspruch 1, wobei, wenn der Winkel an jeder Verbindungsstelle (120) in einem Bereich von 30° bis 90° liegt, eine Metallbedeckung des Stents von 3% bis 15% reicht.

12. Stent nach Anspruch 1, wobei, wenn der Winkel an jeder Verbindungsstelle (120) in einem Bereich von 90° bis 140° liegt, eine Metallbedeckung des Stents von 2% bis 15% reicht.

13. Stent nach Anspruch 1, wobei mindestens einer der verbreiterten Abschnitte (102) einen Hohlraum umfasst, wobei optional:
der verbreiterte Abschnitt (102) 1 bis 10 Hohlräume (105) umfasst; oder
der Hohlraum (105) eine Längsquerschnittsform umfasst, die mindestens eine von einer Bogenform, einer Viereckform und einer Dreiecksform umfasst; oder
der Hohlraum (105) eine Querschnittsform umfasst, die mindestens eine von einer kreisförmigen Form, einer länglichen Form, einer polygonalen Form, einer gewellten Form, einer ringförmigen Form und einer unregelmäßigen Form umfasst; oder
der Hohlraum (105) konfiguriert ist, um darin ein Arzneimittel oder ein strahlundurchlässiges Mittel einzufüllen.

14. Stent nach einem der Ansprüche 1 bis 13, wobei der Stent mindestens zwei Stentgeflechte (100) umfasst, die axial verbunden sind, wobei der Stent optional mindestens eine Verlinkungsstrebe (130) umfasst und wobei die Verbindungsstellen (120) in benachbarten Stentgeflechten (100) durch die Verlinkungsstrebe (130) verbunden sind, wobei die Verlinkungsstrebe (130) optional eine Form umfasst, die mindestens eine von einer linearen Form, einer gewellten Form, einer gezackten Form, einer kreisförmigen Form, einer ringförmigen Form, einer "Ω"-ähnlichen Form und einer "S"-ähnlichen Form umfasst.

15. Mit einem Arzneimittel geladener Stent, der den Stent nach Anspruch 1 umfasst, wobei der mindestens einer der verbreiterten Abschnitte (102) einen Hohlraum (105) umfasst, der konfiguriert ist, um darin ein Arzneimittel einzufüllen.

## Revendications

1. Endoprothèse comprenant au moins une maille d'endoprothèse (100), dans laquelle chaque maille d'endoprothèse (100) comprend une pluralité d'entretoises d'endoprothèse (110) connectées séquentiellement de manière circonférentielle autour de la maille d'endoprothèse (100), dans laquelle la pluralité d'entretoises d'endoprothèse (110) sont connectées séquentiellement bout à bout, et une articulation (120) est formée aux extrémités connectées d'entretoises d'endoprothèse (110) adjacentes, dans laquelle la maille d'endoprothèse (100) est configurée pour se dilater ou se contracter en conséquence de l'élargissement ou du rétrécissement d'angles au niveau des articulations (120),
dans laquelle l'entretoise d'endoprothèse (110) comprend au moins une section principale (101) et au moins une section élargie (102) agencée en alternance avec l'au moins une section principale (101), dans laquelle la section principale (101) présente une largeur inférieure à une largeur de la section élargie (102) ; et dans laquelle, dans chaque maille d'endoprothèse (100), les sections élargies (102) d'entretoises d'endoprothèse (110) adjacentes sont décalées le long d'un axe du treillis d'endoprothèse (100) ;
**caractérisée en ce que** la résistance radiale de l'endoprothèse varie de 1 kPa à 300 kPa ; et
**en ce que**, lorsque l'endoprothèse est dans une configuration dilatée avec un diamètre de 2,0 mm à 5,0 mm, la résistance radiale de l'endoprothèse varie de 1 kPa à 30 kPa.

2. Endoprothèse selon la revendication 1, dans laquelle, lorsque l'angle au niveau de chaque articulation (120) est minimisé, la résistance radiale de l'endoprothèse varie de 50 kPa à 300 kPa.

3. Endoprothèse selon la revendication 1, dans laquelle, lorsque l'angle au niveau de chaque articulation (120) est minimisé, la résistance radiale de l'endoprothèse varie de 100 kPa à 200 kPa.

4. Endoprothèse selon la revendication 1, dans laquelle, lorsque l'endoprothèse est dans la configuration dilatée avec un diamètre de 2,0 mm à 5,0 mm, la résistance radiale de l'endoprothèse varie de 1 kPa à 15 kPa, éventuellement dans laquelle, lorsque l'endoprothèse est dans la configuration dilatée avec un diamètre de 2,0 mm à 5,0 mm, la résistance radiale de l'endoprothèse varie de 1 kPa à 12 kPa.

5. Endoprothèse selon la revendication 1, dans laquelle l'angle au niveau de chaque articulation (120) varie de 0° à 140° ; ou
dans laquelle chaque entretoise d'endoprothèse (110) comprend deux sections principales (101) et une section élargie (102) située entre les deux sections principales (101) ; ou
dans laquelle la maille d'endoprothèse (100) comprend 8 à 24 entretoises d'endoprothèse (110) ; ou
dans laquelle, lorsque l'angle au niveau de chaque articulation (120) est minimisé, dans des entretoises d'endoprothèse (110) adjacentes connectées au niveau d'une même articulation (120), la section élargie (102) de l'une des entretoises d'endoprothèse (110) ne chevauche pas la section principale (101) de l'autre des entretoises d'endoprothèse (110) ; ou
dans laquelle, dans au moins une entretoise d'endoprothèse (110), la section élargie (102) présente des marges d'une même largeur ou de largeurs différentes au-delà de la section principale (101) au niveau de côtés opposés de l'entretoise d'endoprothèse (110) suivant une direction longitudinale de celle-ci ; ou
dans laquelle, dans au moins une entretoise d'endoprothèse (110), la section élargie (102) est affleurante avec la section principale (101) d'un côté de l'entretoise d'endoprothèse (110) suivant une direction longitudinale de celle-ci, éventuellement dans laquelle, dans une configuration dilatée de l'endoprothèse, des entretoises d'endoprothèse (110) adjacentes connectées au niveau d'une même articulation (120) forment une structure en forme de V, dans laquelle les côtés des entretoises d'endoprothèse (110) adjacentes, au niveau desquels les sections principales (101) sont affleurantes avec les sections élargies (102), sont situés simultanément sur un côté intérieur ou un côté extérieur de la structure en forme de V.

6. Endoprothèse selon la revendication 1, dans laquelle, lorsque l'angle au niveau de chaque articulation (120) est dans une plage de 0° à 5°, une couverture métallique de la maille d'endoprothèse (100) varie de 30 % à 99 %.

7. Endoprothèse selon la revendication 1, dans laquelle, lorsque l'angle au niveau de chaque articulation (120) est dans une plage de 5° à 30°, une couverture métallique de la maille d'endoprothèse (100) varie de 5 % à 90 %; ou
dans laquelle, lorsque l'angle au niveau de chaque articulation (120) est dans une plage de 30° à 90°, une couverture métallique de la maille d'endoprothèse (100) varie de 4 % à 15 %, éventuellement dans laquelle, lorsque l'angle au niveau de chaque articulation (120) est dans la plage de 30° à 90°, la couverture métallique du treillis d'endoprothèse (100) varie de 8 % à 15 %.

8. Endoprothèse selon la revendication 1, dans laquelle, lorsque l'angle au niveau de chaque articulation (120) est dans une plage de 90° à 140°, une couverture métallique de la maille d'endoprothèse (100) varie de 3 % à 12 %.

9. Endoprothèse selon la revendication 1, dans laquelle, lorsque l'angle au niveau de chaque articulation (120) est dans une plage de 0° à 5°, une couverture métallique de l'endoprothèse varie de 20 % à 60 .

10. Endoprothèse selon la revendication 1, dans laquelle, lorsque l'angle au niveau de chaque articulation (120) est dans une plage de 5° à 30°, une couverture métallique de l'endoprothèse varie de 5 % à 45 %.

11. Endoprothèse selon la revendication 1, dans laquelle, lorsque l'angle au niveau de chaque articulation (120) est dans une plage de 30° à 90°, une couverture métallique de l'endoprothèse varie de 3 % à 15 %.

12. Endoprothèse selon la revendication 1, dans laquelle, lorsque l'angle au niveau de chaque articulation (120) est dans une plage de 90° à 140°, une couverture métallique de l'endoprothèse varie de 2 % à 15 %.

13. Endoprothèse selon la revendication 1, dans laquelle au moins une des sections élargies (102) comprend une cavité, éventuellement dans laquelle :
la section élargie (102) comprend 1 à 10 cavités (105) ; ou
la cavité (105) comprend une forme en coupe longitudinale comprenant au moins l'une parmi une forme arquée, une forme quadrilatérale et une forme triangulaire ; ou
la cavité (105) comprend une forme en coupe transversale comprenant au moins l'une parmi une forme circulaire, une forme allongée, une forme polygonale, une forme ondulée, une forme annulaire et une forme irrégulière ; ou
la cavité (105) est configurée pour qu'un médicament ou un agent radio-opaque soit rempli dans celle-ci.

14. Endoprothèse selon l'une quelconque des revendications 1 à 13, dans laquelle l'endoprothèse comprend au moins deux mailles d'endoprothèse (100) qui sont connectées axialement, éventuellement dans laquelle l'endoprothèse comprend au moins une entretoise de liaison (130), et dans laquelle les articulations (120) dans des mailles d'endoprothèse (100) adjacentes sont connectés par l'intermédiaire de l'entretoise de liaison (130), éventuellement dans laquelle l'entretoise de liaison (130) comprend une forme comprenant au moins l'une parmi une forme linéaire, une forme ondulée, une forme dentelée, une forme circulaire, une forme annulaire, une forme de type « Ω » et une forme de type « S ».

15. Endoprothèse chargée de médicament, comprenant l'endoprothèse selon la revendication 1, dans laquelle l'au moins une des sections élargies (102) comprend une cavité (105) configurée pour qu'un médicament soit rempli dans celle-ci.
